**Europäisches Patentamt**

**European Patent Office**

**Office européen des brevets**

(19)

(11) Numéro de publication : **0 430 788 A1**

## (12) DEMANDE DE BREVET EUROPEEN

(21) Numéro de dépôt : **90403340.4**

(51) Int. Cl.⁵ : **A61F 2/26**

(22) Date de dépôt : **26.11.90**

(30) Priorité : **28.11.89 FR 8915620**

(43) Date de publication de la demande :
**05.06.91 Bulletin 91/23**

(84) Etats contractants désignés :
**DE ES GB IT**

(71) Demandeur : **Subrini, Louis**
**27 Boulevard Suchet**
**F-75016 Paris (FR)**

(72) Inventeur : **Subrini, Louis**
**27 Boulevard Suchet**
**F-75016 Paris (FR)**

(74) Mandataire : **Picard, Jean-Claude Georges et al**
**Cabinet Plasseraud 84, rue d'Amsterdam**
**F-75009 Paris (FR)**

(54) **Implant de remplissage pénien.**

(57)    Implant de remplissage pénien se présentant sous la forme d'un corps (1) allongé en matière synthétique, d'une épaisseur en principe supérieure à environ 10 mm, caractérisé en ce qu'il est sensiblement plus flexible et plus mou, en permanence et en tout point de sa masse, que les prothèses connues, de sorte à éviter de conférer à la verge une rigidité permanente tout en étant propre à constituer un élément de remplissage total ou subtotal des corps caverneux, en fonction de la longueur propre à chaque individu.

FIG.2.

EP 0 430 788 A1

## IMPLANT DE REMPLISSAGE PENIEN

Jusqu'à maintenant, on a tenté de remédier à certains cas d'impuissance (ceux qui avaient surtout des causes organiques) par des moyens "mécaniques" tels que les prothèses introduites par une opération chirurgicale dans les corps caverneux de la verge. Plusieurs types de prothèses ont été développés, qui ont tous pour but essentiel de conférer à la verge, en permanence ou temporairement, une rigidité artificielle.

Certaines prothèses à rigidité permanente sont en une matière synthétique relativement rigide et s'étendent dans tout ou partie des corps caverneux. Certaines comportent sur pratiquement la moitié de leur longueur une partie proximale relativement peu rigide destinée à occuper la zone périnéale des corps caverneux, et une partie d'extrémité ou distale plus rigide, faisant suite à la première et occupant toute la partie pénienne des corps caverneux.

D'autres prothèses en matière synthétique sont pliables, comportant intérieurement, à cet effet, une tresse de fils d'argent ou analogue ; l'inconvénient de ces prothèses réside dans la raideur qu'elles confèrent à la verge, et dans une rupture relativement rapide de la tresse, par suite des pliages successifs auxquels elle est soumise, ce qui nécessite alors leur remplacement.

La rigidité permanente conférée à la verge, et en particulier à sa partie distale, par tous ces systèmes, représente un inconvénient évident.

Il existe aussi une grande variété de prothèses à rigidité temporaire. Elles comportent en général une cavité interne gonflable par un fluide sous pression, dont le réservoir est soit contenu également dans la prothèse et peut être mis en service par une pression exercée sur elle depuis l'extérieur, soit disposé extérieurement, étant alors implanté à proximité dans le corps du sujet et relié aux cavités en question par des conduites de fluide.

Ces prothèses sont composites, comportent des mécanismes qui peuvent à la longue se détériorer, même s'ils sont simples, et sont évidemment d'un emploi peu agréable.

Le but de la présente invention est de remédier à tous ces inconvénients de la technique antérieure.

L'invention part de la constatation surprenante selon laquelle la seule présence d'un corps de remplissage même non rigide ―de volume suffisant― dans les corps caverneux suffit, au moins dans les cas où l'impuissance n'est pas absolue, à permettre une érection pratiquement naturelle.

Comme on le sait, l'érection commence par un afflux de sang par l'artère caverneuse, ce qui gonfle les sinus caverneux ; lorsqu'ils sont suffisamment gonflés, le réseau veineux sous-albuginéal se trouve assez comprimé pour empêcher le sang d'être évacué, ce qui maintient la rigidité des corps caverneux.

L'explication du phénomène constaté lorsqu'un implant d'un certain volume est introduit dans les corps caverneux est donc que cet implant, occupant une partie relativement importante du volume normal des sinus caverneux, une beaucoup plus faible partie de ce volume est susceptible d'être alimentée par le sang de l'artère caverneuse ; ainsi, les sinus restants, situés autour de l'implant, pourront être beaucoup plus rapidement remplis de sang et pourront-ils à nouveau comprimer le réseau veineux sous-albuginéal, ce qui procurera et maintiendra une érection pratiquement naturelle.

Ces constatations montrent qu'il était en réalité tout à fait inutile d'employer dans tous les cas des prothèses rigides, puisqu'un implant relativement mou permet d'obtenir aussi bien le résultat qui vient d'être indiqué, sans l'inconvénient entraîné auparavant par les prothèses à rigidité permanente.

Un implant pénien conforme à l'invention qui, comme les prothèses connues, se présentera généralement sous la forme d'un corps allongé en matière synthétique, d'une épaisseur en principe égale ou supérieure à 10 ou 13 mm, se caractérisera donc essentiellement en ce qu'il est sensiblement plus flexible et plus mou, en permanence et en tout point de sa masse au moins dans le segment pénien ou distal de l'implant, que les prothèses connues, de sorte à éviter de conférer obligatoirement à la verge une rigidité permanente, tout en étant propre à constituer un élément de remplissage total ou subtotal des corps caverneux, en fonction de la longueur propre à chaque individu.

Comme matière synthétique, on pourra utiliser toute matière appropriée tolérée par les tissus, et en particulier le silicone.

En considération du but recherché tel qu'il a été indiqué plus haut, cette matière pourra présenter avantageusement, en permanence et en tout point de sa masse au moins dans le segment pénien ou distal de l'implant, un module d'élasticité inférieur à environ 50 kg/cm$^2$ pour un allongement relatif de 100%, ce module étant préférentiellement inférieur à 35 kg/cm$^2$, et pouvant même descendre dans certains cas jusqu'à 5 kg/cm$^2$, toujours pour 100% d'allongement relatif, ce module d'élasticité étant, pour les matières plastiques concernées, assez bien représentatif de la flexibilité du matériau.

A titre de comparaison, les prothèses à rigidités différenciées dont il a été question plus haut peuvent avoir un module d'élasticité (toujours pour 100% d'allongement relatif) de l'ordre de 50 à 53 kg/cm$^2$ pour la partie distale la plus rigide, et pouvant descendre jusqu'à 6 kg/cm$^2$ pour la partie la plus souple de la prothèse.

Un autre paramètre permettant de distinguer un implant conforme à l'invention des prothèses de l'art antérieur est la dureté du matériau qui le constitue.

La matière synthétique utilisée pourra présenter avantageusement, également en permanence et en tout point de sa masse au moins dans le segment pénien ou distal de l'implant, une dureté inférieure à environ 70 shore A, tout en restant supérieure à environ 5 shore A. Préférentiellement cette dureté sera de l'ordre d'environ 35 shore A.

A titre de comparaison, les prothèses à rigidités différenciées dont il a été question plus haut peuvent avoir une dureté de 65 à 75 shore A environ pour la partie distale la plus rigide, et de 30 à 40 shore A pour la partie la plus souple.

Il est bien entendu qu'aucune des prothèses de la technique antérieure ne possède en tout point de sa masse et en particulier pour le segment distal, un module d'élasticité ou une dureté permanente aussi faibles que ceux qui ont été mentionnés ci-dessus pour un implant conforme à l'invention.

D'autres caractéristiques d'implants conformes à l'invention, notamment quant à leur forme et à leur structure, vont maintenant être décrites ci-dessous avec référence aux figures du dessin annexé dans lequel :

    - la figure 1 représente un implant conforme à l'invention, propre à être coupé à la longueur voulue ;

    - les figures 2 et 3 montrent deux formes différentes ;

    - les figures 4 à 6 montrent différentes formes de sections transversales ;

    - les figures 7 et 8 montrent des implants conformes à l'invention, à cavités internes ; et

    - la figure 9 montre encore un autre type d'implant à cavité interne.

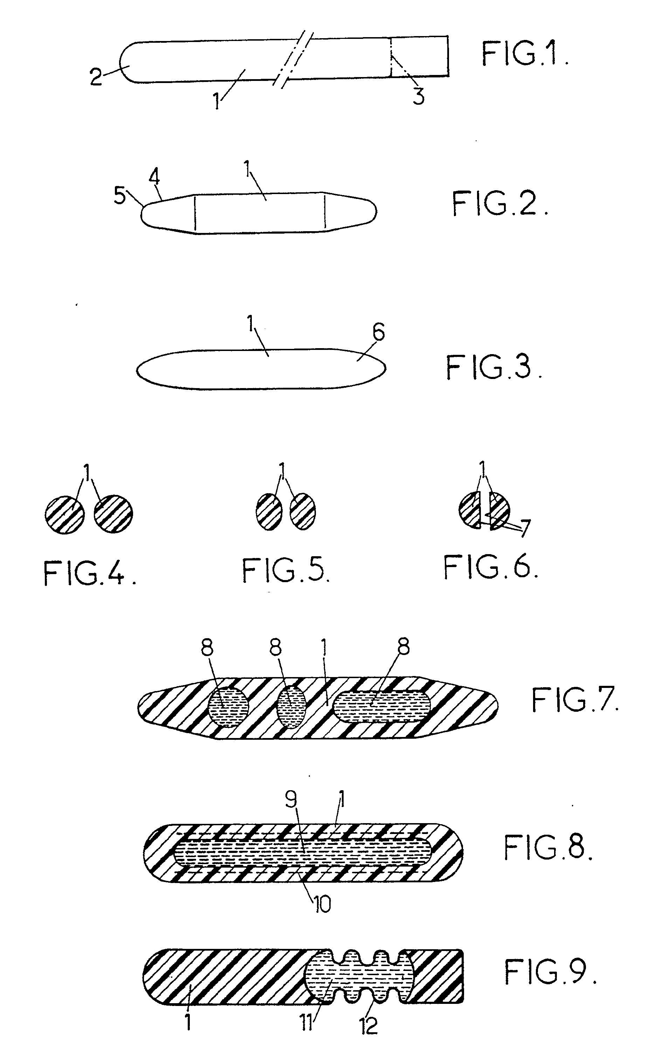

Sur la figure 1 on a représenté une longueur quelconque d'un corps cylindrique et rectiligne 1 en silicone, à section transversale constante et de 10 à 13 et même 14 ou 15 mm de diamètre, dont une seule extrémité, 2, est arrondie en portion de sphère. En coupant ce corps comme indiqué en 3, on peut donc obtenir un implant de la longueur voulue. L'extrémité 3 peut être légèrement arrondie avec un instrument tranchant, pour éviter tout angle vif.

Aux figures 2 et 3 on a représenté au contraire des implants de longueur prédéterminée dont le corps cylindrique 1 se termine par un tronc de cône 4 et une partie sphérique 5 (figure 2) ou se termine en ogive 6 à pointe légèrement arrondie (figure 3). Les implants des figures 2 et 3 ont leurs deux extrémités symétriques l'une de l'autre par rapport au plan transversal médian du corps 1.

Aux figures 4, 5 et 6 on a représenté en section transversale deux implants supposés disposés l'un à côté de l'autre dans les corps caverneux de la verge. La section peut être circulaire (figure 4) ou ovale (ou elliptique) comme visible sur la figure 5.

Les corps 1 peuvent également comporter des aplatissements latéraux 7, les aplatissements des corps implantés dans les deux corps caverneux de la verge pouvant ainsi se faire face (figure 6).

Il est à noter que dans le cas d'un implant de remplissage subtotal, on pourra le laisser se déplacer dans chacun des corps caverneux, à supposer qu'un passage axial y ait été ménagé, ou on pourra au contraire le fixer en un emplacement déterminé, avantageusement la zone périnéale, par une suture ou tout autre moyen.

En tout cas, les formes arrondies des extrémités qui ont été décrites ci-dessus sont bien appropriées, puisqu'elles correspondent à peu près à la forme des extrémités des corps caverneux.

Aux figures 7 et 8 on a montré la possibilité de ne pas utiliser des corps 1 pleins et homogènes comme dans le cas des figures 1 à 6, mais des corps 1 à cavités internes 8 de formes variées (figure 7) ou à cavité interne unique 9 (figure 8).

Les cavités 8 de la figure 7 ont différentes formes que l'on peut rencontrer séparément sur différents implants, mais on comprend que sur un seul et même implant elles auront en principe toutes la même forme ; ce n'est que pour ne pas multiplier le nombre des figures que l'on a représenté diverses formes sur la seule figure 7 ; en tout cas, les cavités peuvent éventuellement communiquer entre elles et être remplies, soit lors de l'implantation soit postérieurement, par un fluide sous pression quelconque, tel que du gel de silicone, ce qui permet d'ajuster les dimensions de l'implant ou ses caractéristiques de flexibilité ou de dureté.

On peut choisir en outre la forme de la ou des cavités et la direction de leur allongement en fonction de la dimension que l'on souhaite augmenter (section ou longueur de l'implant).

Avec de tels implants, comme avec celui de la figure 8, on peut procéder à une expansion progressive d'un pénis de dimensions insuffisantes, en augmentant progressivement les dimensions des implants, ceci sans avoir besoin d'opérer pour les extraire et les remplacer. En effet, le fluide sous pression peut être commodément injecté dans les cavités internes de l'implant à l'aide d'une aiguille, un dispositif d'auto-fermeture très simple empêchant ensuite ce fluide de s'échapper, à moins qu'il ne soit ponctionné pour une raison quelconque, par exemple pour diminuer les dimensions de l'implant.

Il est à noter aussi que la présence de cavités, remplies ou non d'un fluide, à l'intérieur de l'implant, permet de lui donner une dureté inférieure à celle qu'il présenterait s'il était constitué d'un corps plein et homogène ; on peut ainsi diminuer sa dureté en-deçà des valeurs minimales qu'il est possible d'obtenir actuellement avec le silicone médical normal, dont la dureté minimale est de 20 shore A.

Dans une autre variante conforme à l'invention, on peut aussi prévoir de noyer dans la matière synthétique constituant le corps, autour de la cavité, une armature inextensible 10 telle qu'un grillage, un tissu ou analogue, formant autour de la cavité une gaine ou une spirale, pour empêcher une expansion de la cavité même quand du fluide sous pression y est injecté. On a ainsi une possibilité supplémentaire, qui est d'augmenter la dureté de l'implant sans en augmenter les dimensions. L'implant est donc adaptable à tous les cas.

On peut mentionner encore que l'implant conforme à l'invention peut encore se distinguer de toutes les prothèses connues, dont le diamètre ne dépasse jamais 13 mm. Un implant conforme à l'invention, dès lors qu'il n'est pas rigide, peut présenter en effet un diamètre ou une épaisseur sensiblement supérieurs, à savoir de l'ordre de 14 et même 15 mm.

Dans le mode de réalisation de la figure 9, l'implant 1 comprend une cavité 11 dont la paroi comporte une partie en soufflet 12. L'injection d'un fluide sous pression dans la cavité provoquera un allongement progressif de la verge. En augmentant cette pression en plusieurs étapes, on pourra obtenir un allongement ou expansion tissulaire totale importante, l'implant pouvant s'allonger lui-même dans des proportions notables et provoquant l'allongement de la verge par la pression interne qu'il exerce sur le gland.

Si l'on veut porter remède à la fois à une impuissance et à une longueur de verge trop réduite, on pourra laisser cet implant en place en permanence. S'il n'y a pas d'impuissance, on pourra retirer cet implant dès que la verge aura acquis la longueur voulue.

Il est à noter enfin qu'un implant conforme à l'invention, dans toutes ses variantes, pourra être beaucoup plus court que la verge et donc être implanté seulement, soit dans la partie proximale, soit dans la partie distale de celle-ci, selon les besoins.

## Revendications

1. Implant de remplissage pénien se présentant sous la forme d'un corps (1) allongé en matière synthétique, d'une épaisseur en principe égale ou supérieure à environ 10 mm, caractérisé en ce que ladite matière synthétique présente en permanence et en tout point de sa masse au moins dans le segment pénien ou distal de l'implant, un module d'élasticité inférieur à environ 50 kg/cm2 pour un allongement relatif de 100%, et une dureté comprise entre environ 5 et 70 shore A.

2. Implant selon la revendication 1, caractérisé en ce que ladite matière synthétique présente en permanence et en tout point de sa masse au moins dans le segment pénien ou distal de l'implant, un module d'élasticité compris entre environ 5 et 35 kg/cm2 pour un allongement relatif de 100%, et une dureté de l'ordre de 35 shore A environ.

3. Implant selon la revendication 1 ou 2, caractérisé en ce que ladite matière synthétique est du silicone.

4. Implant selon la revendication 1 ou 2, caractérisé en ce que ledit corps (1) est rectiligne ou pratiquement rectiligne et à section transversale constante.

5. Implant selon la revendication 4, caractérisé en ce que la section transversale dudit corps (1) présente un aplatissement (7) latéral, les aplatissements (7) des corps (1) implantés dans les deux corps caverneux de la verge pouvant ainsi se faire face.

6. Implant selon l'une quelconque des revendications 1 à 4, caractérisé en ce que lesdites extrémités (2, 5) du corps (1) ont la forme d'un tronc de cône (4) terminé par une partie sphérique (5).

7. Implant selon l'une quelconque des revendications 1 à 4, caractérisé en ce que lesdites extrémités (2, 5) du corps (1) ont la forme d'une ogive (6).

8. Implant selon l'une quelconque des revendications précédentes, caractérisé en ce que les extrémités dudit corps (1) sont symétriques l'une de l'autre par rapport au plan transversal médian du corps.

9. Implant selon la revendication 1 ou 2, caractérisé en ce que ledit corps comporte intérieurement une ou plusieurs cavités (8, 9) propres à être remplies d'un fluide sous pression.

10. Implant selon la revendication 9, caractérisé en ce qu'une armature inextensible (10) est noyée dans la matière synthétique, autour de ladite cavité, par suite de quoi l'injection d'un fluide dans la cavité permet d'augmenter la dureté de l'implant sans en augmenter les dimensions.

11. Implant selon la revendication 9, caractérisé en ce qu'il comporte une cavité (11) à paroi latérale (12) en forme de soufflet.

FIG.1.

FIG.2.

FIG.3.

FIG.4.     FIG.5.     FIG.6.

FIG.7.

FIG.8.

FIG.9.

Office européen
des brevets

**RAPPORT DE RECHERCHE EUROPEENNE**

Numero de la demande

EP 90 40 3340

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|-----------|---------------------------------------------------------------------------------|-------------------------|--------------------------------------|
| X | US-A-4 483 331 (TRICK)<br>* Abrégé; figures; colonne 3, lignes 2-9,30-38 * | 1-4 | A 61 F 2/26 |
| Y | | 5-10 | |
| Y | US-A-4 589 405 (HEMMETER)<br>* Figure 1; abrégé * | 5 | |
| Y | US-A-4 201 202 (FINNEY)<br>* En entier * | 6-10 | |
| A | GB-A-2 160 777 (TRICK)<br>* Page 2, lignes 8-37; figures * | 10,11 | |
| A | US-A-4 671 261 (FISCHELL) | | |
| A | US-A-4 449 520 (PALOMAR) | | |

**DOMAINES TECHNIQUES RECHERCHES (Int. Cl.5)**

A 61 F

Le présent rapport a été établi pour toutes les revendications

| Lieu de la recherche | Date d'achèvement de la recherche | Examinateur |
|----------------------|-----------------------------------|-------------|
| LA HAYE | 04-03-1991 | STEENBAKKER J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................
& : membre de la même famille, document correspondant

EPO FORM 1503 03.82 (P0402)